# EUROPÄISCHE PATENTSCHRIFT

(11) **EP 1 622 527 B1**
(45) Veröffentlichungstag und Bekanntmachung des Hinweises auf die Patenterteilung: **20.09.2006**
(21) Anmeldenummer: 04730986.9
(22) Anmeldetag: 04.05.2004
(51) Int. Cl.: A61B 17/72

(54) **IMPLANTAT MIT EINEM FIXIERKÖRPER FÜR EINE KNOCHENSCHRAUBE**
IMPLANT COMPRISING A FIXING BODY FOR A BONE SCREW
IMPLANT POURVU D'UN ELEMENT DE BLOCAGE POUR UNE VIS A OS

(30) Priorität: 09.05.2003 DE 10320855
(43) Veröffentlichungstag der Anmeldung: 08.02.2006
(73) Patentinhaber: Aesculap AG & Co. KG, 78532 Tuttlingen (DE)
(72) Erfinder: SAUERESSIG, Thomas, 78532 Tuttlingen (DE); STEDTFELD, Hans-Werner, 90475 Nürnberg (DE)
(74) Vertreter: Boehme, Ulrich
(86) Internationale Anmeldenummer: PCT/EP2004/004710
(87) Internationale Veröffentlichungsnummer: WO 2004/098424

(56) Entgegenhaltungen:
- DE-U- 20 300 987
- DE-U- 20 307 265
- FR-A- 2 701 833
- US-A1- 2001 012 939

## Beschreibung

Die Erfindung betrifft ein Implantat mit einer Gewindebohrung zur Aufnahme einer in diese eingeschraubten Knochenschraube.

Implantate werden an Knochen häufig mittels Knochenschrauben festgelegt, die selbst ein Gewinde zur Aufnahme der Knochenschraube tragen. Die Knochenschraube wird dann sowohl in das Implantat als auch in den Knochen eingeschraubt. Dies kann beispielsweise zur Fixierung von Knochenbruchstücken notwendig sein, auf diese Weise soll erreicht werden, daß diverse Knochenbruchstücke über das Implantat für die Heilung fest miteinander verbunden werden. Derartige Implantate können als Knochenplatten ausgeführt werden oder auch als Markraumnägel, die in den Markraum eingesetzt werden und durch die Wand des umgebenden Röhrenknochens hindurch mit den Knochenfragmenten verschraubt werden.

Es hat sich herausgestellt, daß sich die Knochenschrauben während des Heilungsprozesses lockern können. Dies geschieht insbesondere in der Rehabilitationsphase durch Bewegungen, die ein Patient ausführt. Dabei können sich die Knochenschrauben in der Gewindebohrung leicht verkippen, da immer etwas Spiel zwischen der Knochenschraube und der Gewindebohrung vorhanden ist. Auch eine leichte Drehbewegung ist möglich, und diese kann zum Ausdrehen der Knochenschraube führen.

Das Dokument DE 203 00 987 U1 offenbart ein Implantat für die Osteosynthese mit einem Implantatkörper, der mindestens eine Bohrung mit Gewinde aufweist und einer Knochenschraube, die mit dem Gewinde zusammenwirkt, wenn sie zur Befestigung des Implantatkörpers in einen Knochen eingeschraubt wird, wobei die Gewindebohrung eine Ringnut aufweist, deren Durchmesser größer ist als der Gewindeaußendurchmesser und die einen Ring aus verformbarem Material aufnimmt mit einem Innendurchmesser, der kleiner ist als der Außendurchmesser des Gewindes der Knochenschraube.

Es ist Aufgabe der Erfindung, ein gattungsgemäßes Implantat so auszubilden, daß ein derartiges unbeabsichtigtes Lockern oder gar Ausdrehen der Knochenschraube verhindert wird.

Diese Aufgabe wird bei einem Implantat der eingangs beschriebenen Art erfindungsgemäß dadurch gelöst, daß an dem Implantat in Bohrungsrichtung neben der Gewindebohrung ein Fixierkörper gehalten ist, der eine mit der Bohrung fluchtende Öffnung mit einem Durchmesser aufweist, der kleiner ist als der Außendurchmesser der Gewindegänge der Knochenschraube, und daß der Fixierkörper aus einem Material besteht, das so weich ist, daß sich die Gewindegänge der Knochenschraube beim Einschrauben in die Gewindebohrung des Implantates in dieses Material eingraben oder dieses verdrängen und die Gewindebohrung in der Wand eines rohrförmigen Implantates angeordnet und der Fixierkörper als in den Innenraum des rohrförmigen Implantates eingesetzter Stopfen ausgebildet ist.

Eine solche Ausgestaltung ist insbesondere dann günstig, wenn das Implantat die Form eines Markraumnagels hat.

Man stellt mit diesem Fixierkörper sozusagen eine Schraubenbremse her, in der die eingeschraubte Knochenschraube im Preßsitz gehalten wird. Dadurch wird ein Verkippen der Knochenschraube in der Gewindebohrung verhindert, und dies führt auch dazu, daß das Ausdrehen der Knochenschraube bei Körperbewegungen unterbleibt. Die Gewindegänge der Knochenschraube verformen das Material des Fixierkörpers und erhalten auf diese Weise den gewünschten festen Halt in der Gewindebohrung. Dabei ist von Bedeutung, daß die Knochenschraube durch das Einschrauben in die Gewindebohrung beim Einschraubvorgang selbst sicher geführt wird und die Verformung des Fixierkörpers daher in genau definierter Weise vornehmen kann. Es besteht also nicht die Gefahr, daß die Knochenschraube beim Einsetzen durch den Fixierkörper aus der gewünschten Richtung abgelenkt wird, denn diese gewünschte Richtung wird durch die Gewindebohrung des Implantates eindeutig festgelegt.

Der Fixierkörper zeigt vorzugsweise ein gummielastisches Verhalten, so daß er der Knochenschraube elastisch ausweichen kann und sich dann elastisch an diese anlegt.

Er kann beispielsweise aus Kunststoff bestehen.

In dem Fixierkörper können mehrere mit Gewindebohrungen für Knochenschrauben des Implantates fluchtende Öffnungen vorgesehen sein, so daß man mit einem Fixierkörper mehrere Knochenschrauben in ihren Gewindebohrungen festlegen kann.

Die nachfolgende Beschreibung bevorzugter Ausführungsformen der Erfindung dient im Zusammenhang mit der Zeichen der näheren Erläuterung. Es zeigen:
- Figur 1:: eine Seitenansicht eines Markraumnagels mit einer eingedrehten Knochenschraube und einer Schraubenbremse in Form eines in den Markraumnagel eingesetzten Stopfens;
- Figur 2:: eine Schnittansicht längs Linie 2-2 in Figur 1 und
- Figur 3:: eine Teilschnittansicht ähnlich Figur 2 bei einem bekannten Ausführungsbeispiel mit einer Schraubenbremse in Form eines in das Implantat eingesetzten Ringes.

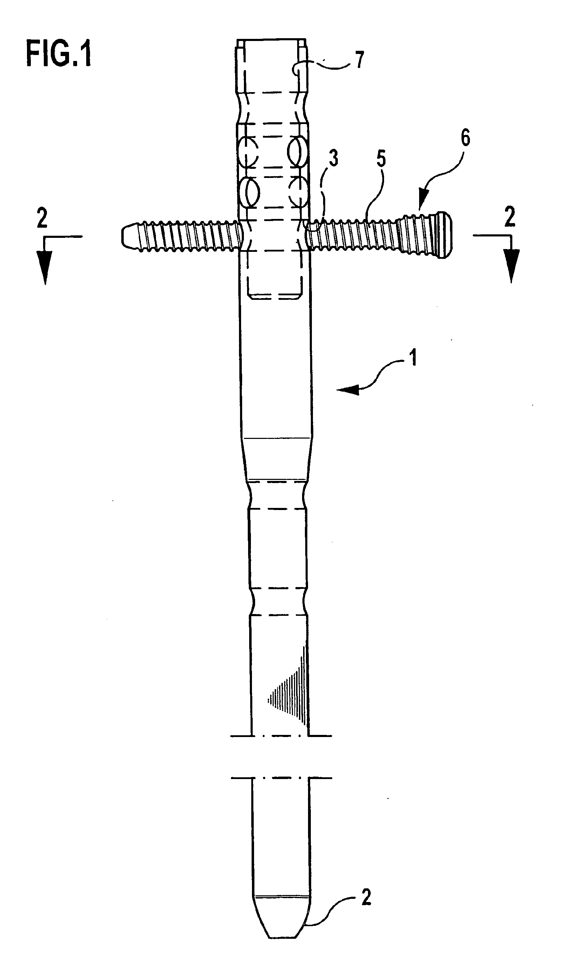

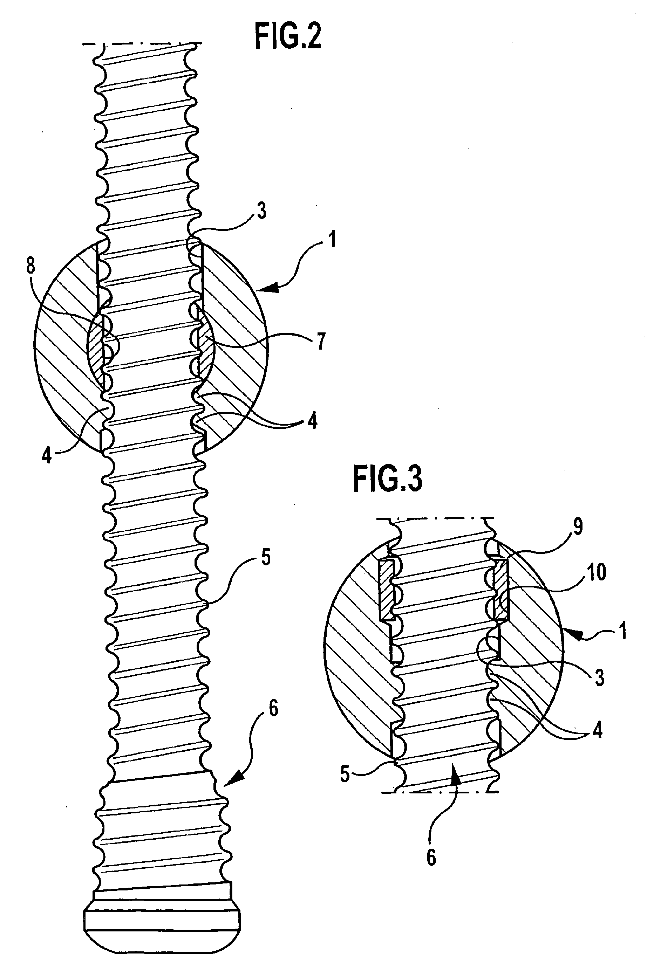

In den Figuren 1 und 2 ist ein Markraumnagel 1 dargestellt, der im wesentlichen rohrförmig ausgebildet ist und mit einer stumpfen Spitze 2 in den Markraum eines Röhrenknochens eingeführt werden kann, um Wandstücke dieses Röhrenknochens über den Markraumnagel 1 zu fixieren. Im rohrförmigen Schaft 3 des Markraumnagels 1 sind mehrere diametrale Durchgangsbohrungen 3 vorgesehen, die an einer Seite des Markraumnagels 1 in der Wand des Markraumnagels 1 Gewindegänge 4 zur Aufnahme der Gewindegänge 5 einer Knochenschraube 6 aufweisen, während derartige Gewindegänge auf der gegenüberliegenden Wandseite des Markraumnagels 1 fehlen, dort hat die Durchgangsbohrung 3 einen Durchmesser, der etwas größer ist als der Außendurchmesser der Knochenschraube 6, so daß diese ohne Einschrauben durch diesen Teil der Durchgangsbohrung 3 hindurchgesteckt werden kann.

Von der Oberseite her ist in den Innenraum des Markraumnagels 1 ein Kunststoffstopfen 7 aus einem gummielastischen Material eingesteckt, der im Bereich der Durchgangsbohrung 3 mit dieser fluchtend eine Durchgangsbohrung 8 aufweist. Der Innendurchmesser dieser Durchgangsbohrung 8 ist kleiner als der Außendurchmesser der Gewindegänge 5 der Knochenschraube 6, so daß sich diese Gewindegänge 5 beim Einschrauben der Knochenschraube 6 in das Material des Kunststoffstopfens 7 eingraben und dieses Material dabei verdrängen. Der Kunststoffstopfen 7 besteht vorzugsweise aus einem elastischen Material, so daß sich das Material nach dem Einschrauben der Knochenschraube 6 wieder elastisch an die Gewindegänge 5 der Knochenschraube 6 anlegt und dadurch die Knochenschraube 6 in der Durchgangsbohrung 3 festlegt. Dadurch wird das Spiel der Knochenschraube 6 in der Durchgangsbohrung 3 verhindert, auch verhindert wird ein unbeabsichtigtes Herausdrehen der Knochenschraube aus den Gewindegängen 4 der Durchgangsbohrung 3.

Im Markraumnagel 1 sind mehrere Durchgangsbohrungen 3 übereinander angeordnet, und jeder dieser Durchgangsbohrungen ist eine entsprechende Durchgangsbohrung im Kunststoffstopfen 7 zugeordnet, so daß in alle Durchgangsbohrungen 3 Knochenschrauben eingeschraubt werden können, die dann durch denselben Kunststoffstopfen 7 in ihrer eingeschraubten Lage fixiert werden.

Während bei dem Ausführungsbeispiel der Figuren 1 und 2 die Knochenschrauben 6 im eingeschraubten Zustand durch eine Schraubenbremse in Form eines Kunststoffstopfens festgelegt werden, wird bei dem an sich bekannten Ausführungsbeispiel der Figur 3 als Schraubenbremse ein Kunststoffring 9 verwendet, der in eine innere Umfangsnut 10 der Durchgangsbohrung 3 eingesetzt ist, zu diesem Zweck ist der Kunststoffring 9 geschlitzt ausgeführt.

Die in Figur 3 dargestellte Ausführung eignet sich besonders für einen massiven Markraumnagel, der also im Bereich der Durchgangsbohrung 3 keinen rohrförmigen Innenraum aufweist. Bei dem dargestellten Ausführungsbeispiel ist zwischen den Gewindegängen 4 der Durchgangsbohrung 3 und der Umfangsnut 10, die den Kunststoffring 9 aufnimmt, ein Abstand vorgesehen, dazwischen umgibt die Durchgangsbohrung 3 die Knochenschraube 6 im Abstand, ohne in die Gewindegänge 5 der Knochenschraube 6 einzugreifen.

Auch bei diesem Ausführungsbeispiel besteht der Kunststoffring 9 vorzugsweise aus einem gummielastischen Material, welches sich an die eingeschraubte Knochenschraube 6 anlegt und die Gewindegänge 5 und damit die Knochenschraube 6 gegen ein Verkippen und Verdrehen sichert.

## Patentansprüche

1. Implantat (1) mit einer Gewindebohrung (3, 4) zur Aufnahme einer in diese eingeschraubten Knochenschraube (6) mit einem in Bohrungsrichtung neben der Gewindebohrung (3, 4) an dem Implantat (1) gehaltenen Fixierkörper (7), der eine mit der Gewindebohrung (3, 4) fluchtende Öffnung mit einem Durchmesser aufweist, der kleiner ist als der Außendurchmesser der Außenkante der Gewindegänge (5) der Knochenschraube (6), wobei der Fixierkörper aus einem Material besteht, das so weich ist, daß sich die Gewindegänge der Knochenschraube (6) beim Einschrauben in die Gewindebohrung (3, 4) des Implantates (1) in dieses Material eingraben oder dieses verdrängen, **dadurch gekennzeichnet, daß** die Gewindebohrung (3, 4) in der Wand eines rohrförmigen Implantates (1) angeordnet und der Fixierkörper (7) als in den Innenraum des rohrförmigen Implantates (1) eingesetzter Stopfen ausgebildet ist.

2. Implantat nach Anspruch 1, **dadurch gekennzeichnet, daß** der Fixierkörper (7) gummielastisches Verhalten zeigt.

3. Implantat nach einem der voranstehenden Ansprüche, **dadurch gekennzeichnet, daß** der Fixierkörper (7) aus Kunststoff besteht.

4. Implantat nach einem der voranstehenden Ansprüche, **dadurch gekennzeichnet, daß** in dem Fixierkörper (7) mehrere mit Gewindebohrungen (3, 4) für Knochenschrauben (6) des Implantates (1) fluchtende Öffnungen (8) vorgesehen sind.

## Claims

1. Implant (1) with a threaded bore (3, 4) for receiving a bone screw (6) screwed into it with a fixing member (7) held on the implant (1) next to the threaded bore (3, 4) in the direction of the bore, said fixing member having an opening aligned with the threaded bore (3, 4) and having a diameter smaller than the external diameter of the outer edge of the thread turns (5) of the bone screw (6), wherein the fixing member consists of a material so soft that the thread turns of the bone screw (6) dig into this material or displace it when the implant (1) is screwed into the threaded bore (3, 4), **characterized in that** the threaded bore (3, 4) is arranged in the wall of a tubular implant (1) and the fixing member (7) is designed as a plug inserted into the interior space of the tubular implant (1).

2. Implant as defined in claim 1, **characterized in that** the fixing member (7) displays elastomeric properties.

3. Implant as defined in any one of the preceding claims, **characterized in that** the fixing member (7) consists of a plastic material.

4. Implant as defined in any one of the preceding claims, **characterized in that** several openings (8) aligned with threaded bores (3, 4) for bone screws (6) of the implant (1) are provided in the fixing member (7).

## Revendications

1. Implant (1) comprenant un alésage fileté ou taraudé (3, 4) destiné à recevoir une vis ou broche à os (6) qui y est vissée, et comprenant un corps de fixation (7) qui est maintenu sur l'implant (1) à côté de l'alésage taraudé (3, 4) dans la direction de l'alésage, et présente une ouverture alignée avec l'alésage taraudé (3, 4) et d'un diamètre qui est inférieur au diamètre extérieur du bord extérieur des filets (5) du filetage de la vis ou broche à os (6), le corps de fixation étant réalisé en un matériau qui est suffisamment mou pour que les filets du filetage de la vis ou broche à os (6), lors du vissage dans l'alésage taraudé (3, 4) de l'implant (1), s'incrustent dans ce matériau ou refoulent celui-ci, **caractérisé en ce que** l'alésage taraudé (3, 4) est agencé dans la paroi d'un implant (1) de forme tubulaire, et le corps de fixation (7) est réalisé sous forme de bouchon inséré dans l'espace intérieur de l'implant (1) de forme tubulaire.

2. Implant selon la revendication 1, **caractérisé en ce que** le corps de fixation (7) présente une propriété d'élasticité du caoutchouc.

3. Implant selon l'une des revendications précédentes, **caractérisé en ce que** le corps de fixation (7) est réalisé en matière plastique.

4. Implant selon l'une des revendications précédentes, **caractérisé en ce que** dans le corps de fixation (7) sont prévues plusieurs ouvertures (8) alignées avec des alésages taraudés (3, 4) pour vis ou broches à os (6) de l'implant (1).
